# EUROPEAN PATENT SPECIFICATION

(11) **EP 3 390 273 B1**
(45) Date of publication and mention of the grant of the patent: **01.09.2021**
(21) Application number: 16828206.9
(22) Date of filing: 01.07.2016
(51) Int. Cl.: B81C 1/00, B29C 43/02, B29C 59/02, B29C 59/04, A61B 17/00, B29L 7/00

(54) **MICROSTRUCTURED SURFACE**
MIKROSTRUKTURIERTE OBERFLÄCHE
SURFACE MICROSTRUCTURÉE

(30) Priority: 17.07.2015 US 201514802632
(43) Date of publication of application: 24.10.2018
(73) Proprietor: Hoowaki, LLC, Greenville, SC 29611 (US); BVW Holding AG, 6330 Cham (CH)
(72) Inventor: HULSEMAN, Ralph A., Greenville, SC 29605 (US); TACKETT, Kenneth N. II, Pendleton, SC 29670 (US); MCPHERSON, Cameron L., Central, SC 29630 (US); HULSEMAN, Carl L., Greenville, SC 29605 (US); TRIP, Roelof, Suwanee, FL 30024 (US); MILBOCKER, Michael, Holliston, MA 01746 (US); BLUECHER, Lucas G., 82547 Eurasburg (DE)
(74) Representative: J A Kemp LLP
(86) International application number: PCT/US2016/040643
(87) International publication number: WO 2017/014936

(56) References cited:
- WO-A1-2013/003373
- WO-A1-2015/074077
- WO-A2-2010/022107
- US-A1- 2005 061 221
- US-A1- 2010 319 183
- US-A1- 2012 011 685
- US-A1- 2013 230 695
- US-A1- 2013 256 944
- US-A1- 2014 131 924
- US-A1- 2014 200 679
- US-B1- 6 368 539
- US-B2- 7 935 428

## Description

The present invention relates to microstructured surfaces, and more particularly to a substrate having increased surface area through a hierarchical arrangement of multiple shapes and sizes of micro features providing a surface that is superhydrophobic while having enhanced sliding friction and adhesion when placed against liquid covered surfaces.

Biomimetics, or the study of systems in nature, has become an increasingly popular source of inspiration to solve complex human issues. In order to create materials with specific properties, many research groups have identified plants or animals in nature that exhibit the desired properties, and then studied the underlying mechanisms.

Studies revolving around plants and their petal effects have shown to portray a superhydrophobic effect, where round droplets that slide easily on the surface are maintained, and various levels of adhesion, where these droplets can stick without rolling off. This effect is particularly notable in rose petals, which has become a focal point of study for transitioning this trait into everyday usage. Many potential areas of usage have been identified, ranging from surgical utilizations to improved comfort and durability of clothing against skin.

The surface of a rose petal is covered by hierarchical micro- and nanostructures, which allow a droplet of water to rest with a high contact angle and a high pinning force. These combined properties have been termed the "rose petal effect" by those skilled in the art. The wetting regime of the rose petal effect is a combination of Cassie-Baxter and Wenzel wetting regimes. (See Cassie, A. and S. Baxter. "Wettability of porous surfaces." Trans. Faraday Soc. 40 (1944), 546-551; and, Wenzel, R. N. "Resistance of solid surfaces to wetting by water." Ind. Eng. Chem. 28 (1936), 988-994.; and, US Pub. No. 2012/0052241). The former describes a non-wetting scenario, while the later describes complete wetting. The microstructures of the rose petal surface are wetted, while the nanostructures are not wetted. This gives the rose petal its notable properties.

Previous efforts in the field have attempted to mimic the natural microstructures found in the rose petal, and also the lotus leaf, by creating a pyramidal structure that somewhat replicates the rose petal effect. The random bumps and microstructures of the rose petal, however, have made it difficult to attempt to manufacture similar arrangements. The pyramidal structures created were 15 micrometers long, 11 micrometers tall, had a center to center to center distance of 20 micrometers, and were designed angularly at 54.7 degrees via stereolithography. Nanostructures were incorporated into these designs that were 150 nanometers in diameter, 150 nanometers tall, and 200 nanometers pitch distance. The limitation using this method is that stereolithography can only create angled structures at a 57 degree angle, thus eliminating the option for forming round microstructured bumps.

Many surfaces using various techniques have been made in attempt to mimic the rose petal's properties. However, the processes used required numerous materials and multiple steps, which is more time consuming and expensive. It is best to minimize the number of steps and time needed in a micro surface molding process to minimize error, decrease large scale production costs, and decrease the time of manufacturing. Using a smaller number of materials also decreases production costs.

A study by Liu et al used an electroforming process as well to mimic several surfaces in nature, including the red rose petal. Their focus was to create an easy one-step process, which they achieved by using a copper substrate, the electrodepositing technique, and a solution containing various compounds. Although their method is simple and fast, it still involves specific measuring and preparation the solution ahead of time, which increases the room for error. This method is also limited by the necessity of a metal that can act as a cathode and electrode in an electrolyte cell. If this surface were to be applied to an implantable medical device, further studies would need to be done to analyze the biocompatibility of the surface and leaching of potentially toxic ions. (Liu, Y., S. Li, J. Zhang, Y. Wang, Z. Han, and L. Ren. "Fabrication of Biomimetic Superhydrophobic Surface with Controlled Adhesion by Electrodeposition." Chemical Engineering Journal 248 (2014), 440-447). Multiple methods of creating surfaces similar to the rose petal are also limited to metal substrates.

US 2005/0061221 discloses a superhydrophobic coating. US 7,935,428 discloses a component with a coating for reducing the wettability of the surface and method for production thereof. WO 2015/074077 discloses articles for manipulating impinging liquids and associated methods. US 2014/0200679 discloses implantable superhydrophobic surfaces. WO 2010/022107 discloses nanostructured superhydrophobic, superoleophobic and/or superomniphobic coatings, methods for fabrication, and applications thereof.

Accordingly, it is an object of the present invention to provide a microstructure surface comprising a substrate with four sets of micro features, wherein each of said sets of micro features cooperate to increase the surface area and affect at least one of adhesion, friction, hydrophilicity and hydrophobicity of the substrate.

The present invention is defined in the claims.

According to the invention, there is disclosed a microstructured surface comprising a substrate having an undulating surface defining a first set of micro features; a second set of micro features disposed on said first set of micro features and selected from the group consisting of microstructured projections and microstructured cavities, and combinations thereof, wherein said second set of micro features is smaller than said first set of micro features; a third set of micro features disposed on at least one of said first set of micro features and said second set of micro features, said third set of micro features selected from the group consisting of microstructured projections and microstructured cavities, and combinations thereof, wherein said third set of micro features is smaller than said second set of micro features; a fourth set of micro features disposed on side surfaces of each microstructure of said second set of micro features, wherein said fourth set of micro features is selected from the group consisting of flutes and ribs; and, said second set and said third set of micro features each including a portion of micro features extends along an axis normal to the curve of said undulating surface; wherein each of said sets of micro features cooperate to increase the surface area and affect at least one of adhesion, friction, hydrophilicity and hydrophobicity of said substrate.

In a further advantageous embodiment, each microstructure of said first, second, third and fourth sets of micro features has a respective pitch, height/depth, and diameter, and wherein said micro features are arranged so that liquids penetrate between at least said first and second sets of micro features in a Wenzel fully wetted state when applied against a liquid covered surface with a water droplet greater than 10 micro litres in size.

In a further advantageous embodiment, said undulating surface defining said first set of micro features includes rounded peaks that facilitate pressure distribution across said substrate.

In a further advantageous embodiment, said second and third sets of micro features are uniformly distributed across said rounded peaks to provide increased surface area to said first set of micro features.

In a further advantageous embodiment, said rounded peaks define areas of increased pressure when said substrate is applied against a liquid covered surface that promote a transition of liquid droplets from a suspended Cassie-Baxter state to a Wenzel fully wetted state among at least said first and second sets of micro features.

In a further advantageous embodiment, said undulating surface comprises a shape selected from the group consisting of rounded sloping projections and rounded sloping cavities forming rounded peaks and rounded valleys that produce a continuously curving surface on at least a portion of said substrate.

In a further advantageous embodiment, a pitch between each of said rounded sloping projections and each of said rounded sloping cavities of said undulating surface is within a range of about 450 microns to about 750 microns to facilitate adhesion of said substrate against a liquid covered surface.

In a further advantageous embodiment, a diameter at a generally circular base of each of said rounded sloping projections and each of said rounded sloping cavities of said undulating surface is within a range of about 450 microns to about 750 microns to facilitate adhesion of said substrate against a liquid covered surface.

In a further advantageous embodiment, a height/depth of each of said rounded sloping projections and each of said rounded sloping cavities of said undulating surface is within a range of about 100 microns to about 500 microns to facilitate adhesion of said substrate against a liquid covered surface.

In a further advantageous embodiment, a pitch between each of said second set of micro features is within a range of about 10 microns to about 50 microns to facilitate adhesion of said substrate against a liquid covered surface.

In a further advantageous embodiment, a diameter of each of said second set of micro features is within a range of about 10 microns to about 50 microns to facilitate adhesion of said substrate against a liquid covered surface.

In a further advantageous embodiment, a height/depth of each of said second set of micro features is within a range of about 10 microns to about 50 microns to facilitate adhesion of said substrate against a liquid covered surface.

In a further advantageous embodiment, a pitch between each of said third set of micro features and each of said fourth set of micro features is within a range of about 1 microns to about 10 microns to facilitate adhesion of said substrate against a liquid covered surface.

In a further advantageous embodiment, a height/depth of each of said third set of micro features and each of said fourth set of micro features is within a range of about 0.4 microns to about 10 microns to facilitate adhesion of said substrate against a liquid covered surface.

The construction designed to carry out the invention will hereinafter be described, together with other features thereof. The invention will be more readily understood from a reading of the following specification and by reference to the accompanying drawings forming a part thereof, wherein an example of the invention is shown and wherein:
Figure 1 shows a magnified view of a substrate according to the present invention;
Figure 2 a magnified view of a further embodiment of the substrate according to the present invention;
Figure 3 shows a schematic side view of a first embodiment of the substrate according to the present invention;
Figure 4 shows a schematic side view of a second embodiment of the substrate according to the present invention;
Figure 5 shows a schematic perspective view of the second, third and fourth sets of micro features according to the present invention;
Figure 6 shows a schematic top view of the second, third and fourth sets of micro features according to the present invention; and,
Figures 7A-7D show various embodiments of the undulating surface of the substrate according to the present invention.

With reference to the drawings, the invention will now be described in more detail. Unless defined otherwise, all technical and scientific terms used herein have the same meaning as commonly understood to one of ordinary skill in the art to which the presently disclosed subject matter belongs. Although any methods, devices, and materials similar or equivalent to those described herein can be used in the practice or testing of the presently disclosed subject matter, representative methods, devices, and materials are herein described.

Unless specifically stated, terms and phrases used in this document, and variations thereof, unless otherwise expressly stated, should be construed as open ended as opposed to limiting. Likewise, a group of items linked with the conjunction "and" should not be read as requiring that each and every one of those items be present in the grouping, but rather should be read as "and/or" unless expressly stated otherwise. Similarly, a group of items linked with the conjunction "or" should not be read as requiring mutual exclusivity among that group, but rather should also be read as "and/or" unless expressly stated otherwise.

Furthermore, although items, elements or components of the disclosure may be described or claimed in the singular, the plural is contemplated to be within the scope thereof unless limitation to the singular is explicitly stated. The presence of broadening words and phrases such as "one or more," "at least," "but not limited to" or other like phrases in some instances shall not be read to mean that the narrower case is intended or required in instances where such broadening phrases may be absent.

Microstructure, micro feature, micro, micron, micrometer as may be used herein denote structures of a size scale of 10⁻⁶.

The present invention taken inspiration from the natural rose petal and its properties to develop a novel multi-level tiered microstructured surface pattern. This surface not only exhibits the unique and sought-after qualities of the rose petal, but significantly enhances them. Further, as detailed herein below, also overcomes various manufacturing and fabrication limitations of rose petal effect surfaces that have previously been developed in the art. The high adhesive and superhydrophobic properties, as well as, the molding process of the microstructured surface defined herein can be used to improve countless machines and devices in a variety of industries, particularly, medical products.

The microstructured surfaces of the present invention have two or more levels of micro objects assembled in a manner to yield a high surface area while maintaining a minimum spacing between objects to allow for liquid flow and penetration (to promote surface adhesion) and while maintaining a minimum structural strength obtained by keeping height to width aspect ratio of all features below a critical level at which materials strength is exceeded.

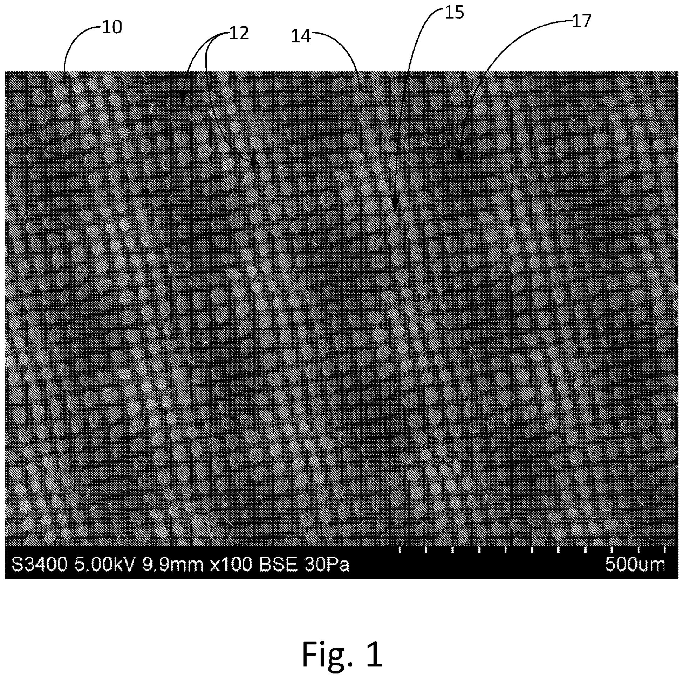

Referring to Figure 1, a first embodiment of a microstructure arrangement according to the present invention is shown comprising a substrate, designated generally as 10. In the illustrated embodiment, substrate 10 has an undulating surface forming a series of rounded peaks and valleys that produce a continuously curving surface across at least a portion of substrate 10. The undulating surface of substrate 10 defines a first set of micro features, designated generally as 12. In Figure 1, substrate 10 is constructed and arranged to focus on a series of rounded knobs forming peaks 15 projected upwardly from the surface with associated valleys 17 disposed between peaks 15.

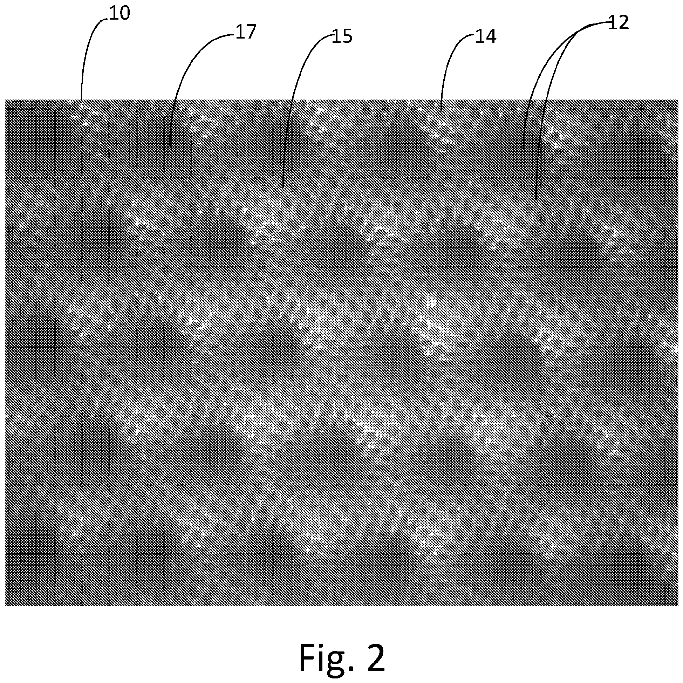

In a second embodiment shown in Figure 2, the inverse arrangement is shown in which substrate 10 is constructed and arranged to focus on a series of rounded cavities forming valleys 17 extending inwardly into substrate 10 as the dominant feature with the associated peaks 15 disposed between valleys 17. In both embodiment, the surface of substrate 10 is continuously curving throughout the undulating surface pattern area.

According the present invention and as illustrated in several of the embodiments, the undulating surface of substrate 10 has a repetitive oscillation of rounded, non-flat curvatures. The curvatures of the substrate surface can be described by mathematical formulas incorporating trigonometric functions sine, cosine, tangent or exponential and power series functions. These mathematical formulas are used in computer aided design and computer aided manufacturing software to create micro surfaces using rapid prototyping, milling, electrical discharge machining or similar techniques to create a polymer or metal surface with an undulating surface forming desired micro features. The advantage of using mathematical formulas is that large numbers of rounded, non-flat features can be created rapidly in computer aided design and computer aided manufacturing software. Micro features of this type cannot be created using lithographic techniques.

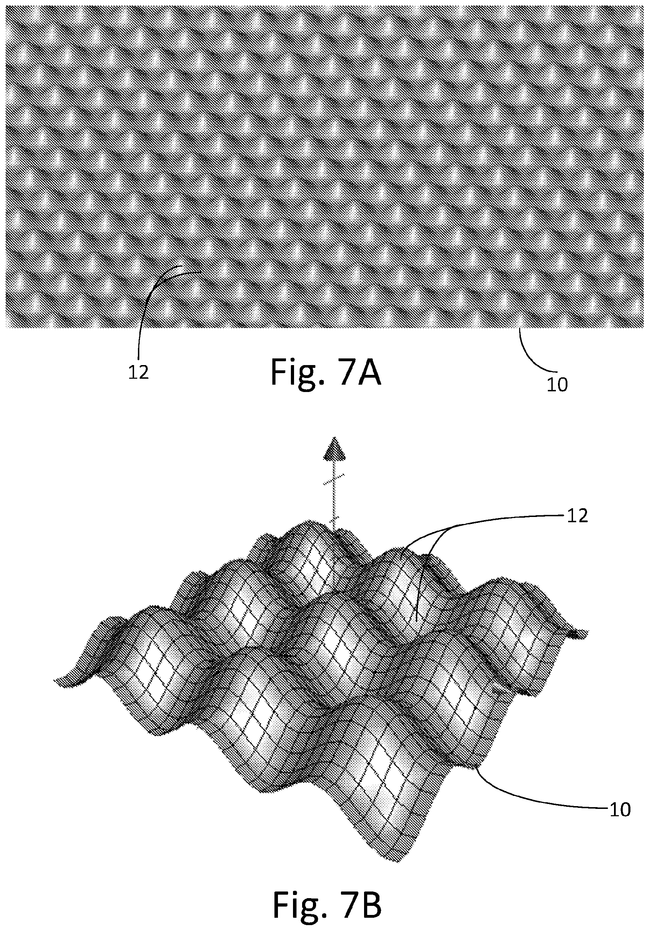

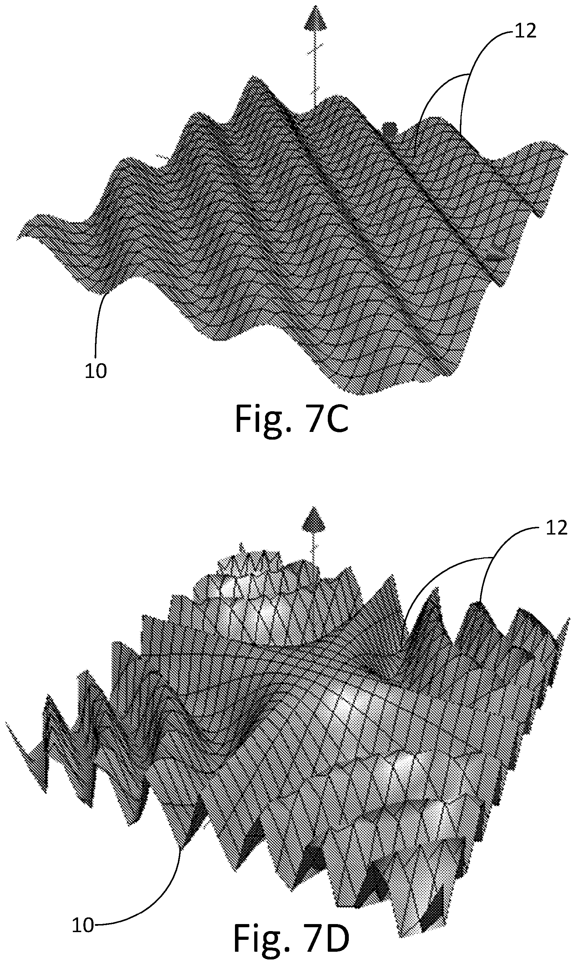

Referring to Figures 7A-7D, a selection of substrates 10 are shown having various undulating surface patterns that provide alternative curved surface micro features across substrate 10. These embodiments are for illustrative purposes only as example embodiments of substrate 10 and are not limiting of the present invention.

According to the present invention, first set of micro features 12 includes dimensions selected from a size within a range of about 100 microns to about 999 microns. More specifically as will be detailed herein below, in a preferred embodiment, the undulating surface is arranged so that first set of micro features 12 has rounded cavities of 750 microns, a pitch of 750 microns, and a depth of about 240 to 500 microns. This arrangement of substrate 10 is intended to promote adhesion to wet surface, specifically organ and muscle tissue for use in surgical materials.

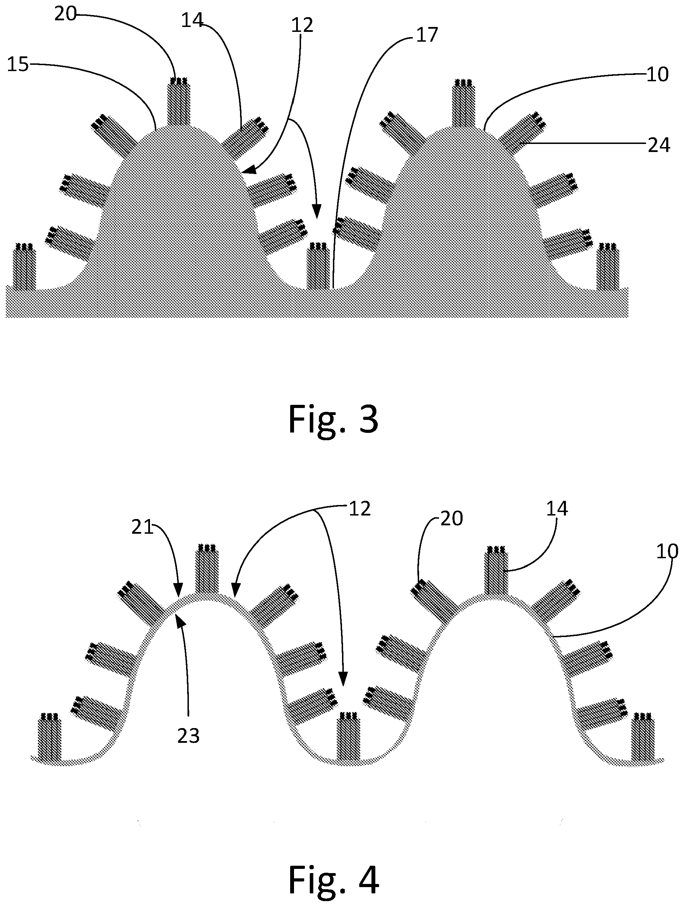

Referring to Figures 3-6, a second set of micro features 14 is disposed on the surface of substrate 10. In one embodiment, second set of micro features 14 is molded on first set of micro features 12 of substrate 10. As detailed herein below, in a preferred embodiment, substrate 10 is a compression molded polymeric material in which first and second sets of micro features 12, 14 are formed on substrate 10 during a single molding step. First and second sets of micro features 12, 14 cooperate to increase the surface area and affect at least one of adhesion, friction, hydrophilicity and hydrophobicity of substrate 10.

Preferably, the compression molded polymeric material forming substrate 10 is selected from the group consisting of PDMS, PMMA, PTFE, PEEK, FEP, ETFE, PTFE, PAEK, polyphenylsulfone, polyurethanes, polyacrylates, polyarylates, thermoplastics, polypropylene, thermoplastic elastomers, fluoropolymers, biodegradable polymers, polycarbonates, polyethylenes, polyimides, polystyrenes, polyvinyls, polyoelefins, silicones, natural rubbers, synthetic rubbers, and combinations thereof. In one preferred embodiment of the present invention, substrate 10 comprises a polylactic acid bioresorbable polymer (PLA).

In the illustrated embodiments, second set of micro features 14 is selected from the group consisting of microstructured projections and microstructured cavities, and combinations thereof. The illustrated embodiment in Figure 2, second set of micro features 14 comprise microstructured cavities extending downwardly into substrate 10. In the illustrated embodiments of Figures 3-6, second set of micro features 14 comprises microstructured projections extending upwardly from substrate 10. Preferably, in the illustrated embodiments of Figures 3-6, the microstructured projections of said second set of micro features 14 comprise generally cylindrical pillars. Preferably, in the illustrated embodiment of Figure 2, the microstructured cavities of second set of micro features 14 comprise generally cylindrical recesses.

Referring to Figure 4, in one embodiment in which substrate 10 is a thin film substrate and has operable opposing top and bottom surfaces, first set of micro features 12 disposed on a top surface 21 of substrate 10 form a complementary shape on a bottom surface 23 of substrate 10 so that a rounded peak on top surface 21 forms a rounded valley on bottom surface 23 and the rounded valley on top surface 21 forms a rounded peak on bottom surface 23.

Referring to Figures 1, 2 and 4, in an embodiment in which substrate 10 is a thin film substrate and has operable opposing top and bottom surfaces, second set of micro features 14 may include a series of microstructured projections on one of top surface 21 or bottom surface 23 of substrate 10, which then define a series of complementary microstructured cavities on the other opposite top surface or bottom surface 21, 23. For example, Fig. 1 can be representative of a thin film substrate embodiment with a top surface having microstructured projections, in which case Fig. 2 is representative of a complementary bottom surface on the thin film substrate wherein the microstructured projections on the top surface in Fig. 1 form microstructured cavities on the opposite bottom surface in Fig. 2. Likewise, in an embodiment in which second set of micro features 14 comprises microstructured cavities which project downwardly through substrate 10 from a top surface 21, they form complementary microstructured projections on the opposing bottom surface 23.

Referring to Figures 1, 3 and 4, in the illustrated embodiments, second set of micro features 14 include at least a portion of micro features that extend along an axis normal to the curve of the undulating surface of substrate 10 at a given point for the individual microstructure. In this way, second set of micro features 14 follow the curvature of first set of micro features 12.

According to the present invention, second set of micro features 14 includes dimensions selected from a size within a range of about 10 microns to about 100 microns.

Further, second set of micro features 14 preferably have a height to width aspect ratio of less than 5, and a minimum spacing of 1 micron between each micro feature of said second set of micro features to maintain structural strength while allowing for liquid flow and penetration between the individual microstructures comprising second set of micro features 14.

Referring to Figures 3-6, a third set of micro features 20 may also be disposed on substrate 10. Preferably, third set of micro features 20 is selected from the group consisting of microstructured projections and microstructured cavities, and combinations thereof. In one embodiment, the microstructured projections of third set of micro features 20 comprise generally cylindrical pillars. Referring to Figure 2, in one embodiment, the microstructured cavities of third set of micro features 20 comprise generally cylindrical recesses.

Preferably, third set of micro features 20 are compression molded simultaneously with first and second sets of micro features 12, 14. In a further preferred embodiment, third set of micro features 20 have a height to width aspect ratio of less than 5, and a minimum spacing of 1 micron between each micro feature of third set of micro features 20 to maintain structural strength while allowing for liquid flow and penetration between said third set of micro features. The aspect ratio is smaller when devices are made of lower strength materials and larger when made form stronger materials. The spacing between features is smaller for less viscous liquids and larger for more viscous liquids.

Referring to Figures 1, 3, 4, in the illustrated embodiments, third set of micro features 20 include at least a portion of micro features that extend along an axis normal to the curve of the undulating surface of substrate 10. For purposes of the present invention in which the second and third sets of micro features 14, 20 extend along an axis normal to the curve of the undulating surface, the normal line to a curve is the line that is perpendicular to the tangent of the curve at a particular point along the curve.

In the illustrated embodiments, second set of micro features 14 is smaller than first set of micro features 12, and third set of micro features 20 is smaller than second set of micro features 14. According to the present invention, third set of micro features 20 includes dimensions selected from a size within a range of about 0.4 micron to about 10 microns.

Referring to Figures 1 and 3-6, in one embodiment, third set of micro features 20 are disposed on an end surface 22 of second set of micro features 14. In a further advantageous embodiment, third set of micro features 20 are disposed on first set of micro features 12 between second set of micro features 14. In a further advantageous embodiment, third set of micro features 20 are disposed on an end surface 22 of second set of micro features 14, as well as, disposed on first set of micro features 12 between second set of micro features 14.

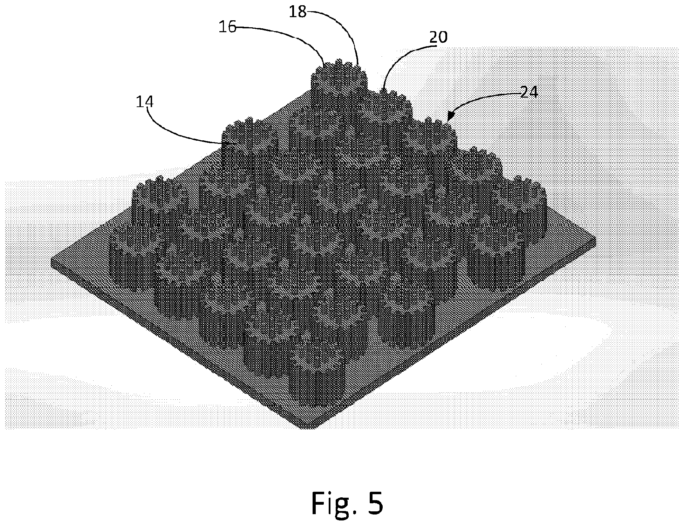

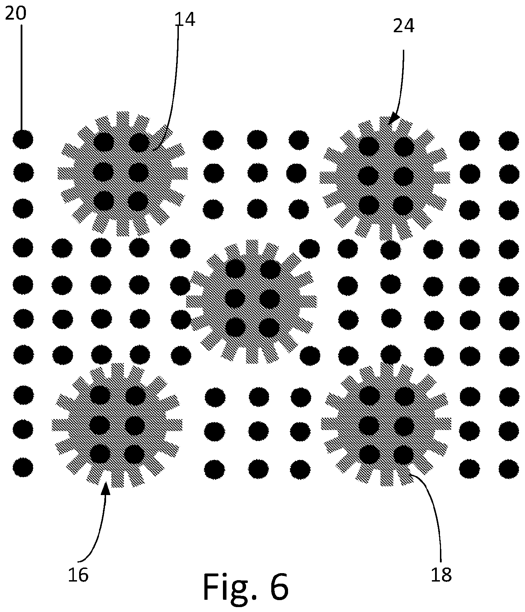

Referring to Figures 5 and 6, a fourth set of micro features 24 may be disposed on side surfaces of second set of micro features 14. Fourth set of micro features 24 is selected from the group consisting of flutes 16 and ribs 18, and combinations thereof. In the illustrated embodiments, flutes and ribs 16, 18 run vertically along the height of the side surfaces on the outside circumference of each microstructure comprising said second set of micro features 14. Fourth set of micro features 24 preferably include dimensions selected from a size within a range of about 0.4 micron to about 10 microns. Preferably, fourth set of micro features 24 are compression molded simultaneously with said first, second, and third sets of micro features into substrate 10. Preferably, flutes and/or ribs 16, 18 with features and spacing larger than 1 micron are added to the exterior of the cylindrical pillars or cavities defining second set of micro features 14 to both add surface area and to increase structural resistance to bending and breaking. The spacing between individual microstructures of fourth set of micro features 24 and between individual microstructures of second set of micro features 14 is smaller for less viscous liquids and larger for more viscous liquids.

Third set of micro features 20 cover both the tops of pillars and bottoms of cavities and the area between the pillars or cavities defining second set of micro features 14 in a substantially uniform manner. Together the second and third sets of micro features 14,20 substantially increase the surface area exposed to the liquid covering the opposite surface from substrate 10.

Depending on the desired application, the first, second, third and fourth sets of micro features cooperate to increase the surface area of substrate 10 to effect at least one of adhesion, friction, hydrophilicity and hydrophobicity of substrate 10. In one embodiment, substrate 10 has a surface adhesion with a sliding friction force of greater than 50 gr/cm2 when applied against moist organ and muscle tissue. In a preferred embodiment, substrate 10 has a surface adhesion with a sliding friction force of about 325 gr/cm2 when applied against moist organ and muscle tissue.

In early studies, rose petal structures were characterized and a 'rolling hill' effect in microstructures was observed. Additionally, smaller microstructures were noted as 'hairs' that seemed to contribute strongly to the superhydrophobic effect. In order to best simulate this scheme, an undulating surface design for a substrate was created as set forth herein that could reproduce and improve upon rounded microstructure effects seen naturally. The initial design focused on an undulating surface having a sinusoidal waveform cross-section with features from 300 microns diameter and pitch of 100 microns. Referring to Figures 3 and 4, in the illustrated embodiments, substrate 10 is shown having an undulating surface in which the curvature of the surface has a sinusoidal waveform cross-section forming a series of rounded peaks 15 and rounded cavities 17.

The dimensions for the third set of micro features 20 include in one embodiment pillars having 3 micrometers diameter, 6 micrometers pitch, and 5 micrometers tall. The second set of micro features 14 in one embodiment includes microstructure pillars that are at least 45 micrometers in diameter, 45 micrometers tall, and 10 micrometers spacing, with fluted side surfaces. When overlapped together, the second and third sets of micro features 14, 20 are formed along an axis normal to the curvature of the undulating surface features. These are also maintained multi-dimensionally over the round features.

To re-create the superhydrophobic effect found in nature with the rose petal, second set of micro features 14 was added with 'fluted' or 'ribbed' features running down the side surface. These fluted and ribbed features that define fourth set of micro features 24 simulate the smaller, hair-like microstructures of the rose petal to further promote hydrophobocity.

Accordingly, each microstructure of said first, second, third and fourth sets of micro features 12, 14, 20 and 24 have a respective pitch, height/depth, and diameter, and wherein are arranged so that liquids penetrate between at least said first and second sets of micro features in a Wenzel fully wetted state when applied against a liquid covered surface to promote adhesion between substrate 10 and the adjacent surface.

Preferably, the undulating surface of first set of micro features 12 includes rounded peaks that facilitate pressure distribution across substrate 10 when pressed against a liquid covered surface. Preferably, second and third sets of micro features 14, 20 are uniformly distributed across the rounded peaks of first set of micro features 12 to provide increased surface area to first set of micro features 12. The rounded peaks define areas of increased pressure when substrate 10 is applied against a liquid covered surface that promote a transition of liquid droplets from a suspended Cassie-Baxter state to a Wenzel fully wetted state among at least said first and second sets of micro features. In a preferred embodiment, first, second and third sets 12, 14, 20 of micro features allow for liquid penetration to a Wenzel fully wetted state, while the fourth set of micro features 24 are constructed and arranged to maintain superhydrophobic characteristics.

The function of the second and third sets of micro features 14, 20 is to create a large surfaces area simultaneously with spacing wide enough the viscous liquids can flow through the structure at low pressure. Low pressure in this application is defined in the context of the weight associated with liquid droplets being sufficient to create a Wenzel fully wetted state to promote adhesion of substrate 10 to an adjacent liquid covered surface. Accordingly, the microstructured surfaces of the present invention are designed to facilitate transitions from a Cassie-Baxter suspended droplet state to the Wenzel fully wetted state with a water droplet of greater than 10 micro liters in size.

One function of the undulating surface of first set of micro features 12 is to further increase the surface area while creating areas of increased pressure at the peaks of the features. These areas of increased surface area wet first, causing a rapid transition from the Cassie-Baxter suspended droplet state to the Wenzel fully wetted state. A second function of the undulating surface of first set of micro features 12 is to keep the peak pressure low enough and to spread the pressure such that there is little or no penetration through the liquid layer on the surface into the underlying material. This is important for example to avoid trauma and inflammation of underlying biological tissue in medical applications.

The second and third sets of micro features 14, 20 are spread uniformly over the undulating surface of first set of micro features 12 and are normal to the curve of the undulating surface. That is they are perpendicular to a surface tangent at each point of the microstructure on surface. This ensures that the maximum surface area is created in a structure that can be molded.

Studies of plants surfaces have shown to portray unique effects such as the Lotus leaf super hydrophobic effect, where water droplets slide from the surface leading to a self-cleaning effect. However, the hierarchical structure of the upper surface of red rose petals, exhibit a different effect. The rose surfaces are super hydrophobic, however water droplets stick to the surface with high adhesion.

A series of friction drag tests were conducted on various multi-level microstructure arrangement for substrate 10 as compared to flat substrates with various sets of micro features. As the pattern numbers increased, undulating surface depth was increased as well, resulting in increased observed adhesion on wet tissue.

To conduct the friction drag tests, PLA 704 dissolved in acetone is used (supplied by MAST as scrap material). Mechanical localization characteristics were assessed. Cutlets of bovine "steak" were purchased and sliced into 3 cm cubes and affixed to a localized platform. The meat was kept well hydrated with physiologic saline solution at 22°C. Test articles were cut to 1 X 1 cm squares and mounted on discs to which was attached the filament through which force would be applied to the test article. Shear was measured by placing the strip on the 3 cm cube of meat and pulling horizontally to the surface. Thus, these measurements yield a force per unit area (1 cm²). In these tests moist meat was used rather than water-immersed to better reflect surgical conditions as an intended application of the present invention. In all measurements, clear outliers were discarded, and the run was repeated with additional test articles. An Instron Mini 55 was used to record force and the crosshead speed was 0.1 cm/sec. The load cell limit was 200 g with an accuracy of +/- 0.1 g. All measurement rounded to nearest gram. All measurements were done with a 0.5 gram disc. All measurements were done with fresh casts to avoid texture filling.

The first test was conducted with a duplicated surface of the red rose petal onto polylactic acid bioresorbable polymer (PLA).

**Table 1 - Friction force of copy of red rose upper surface**

| Texture | Sliding friction force against moist meat (grams force) |
|---|---|
| Positive from organic rose molded in PLA N=3 | 117 +/-15 |
| Smooth silicone surface | < 10 |

The friction force of the copy of the organic rose petal is a level that may be useful for medical, industrial and recreational devices, However, the small size and irregular nature of the rose petal prevents scale up to cover manufacturing equipment. Researchers have reported fabrication of artificial rose petal surfaces created by techniques unsuitable for molding equipment as noted above.

Using techniques described herein, we manufactured the surfaces shown in Table 3.

**Table 3 - Description of micro patterns 067A, 068A, 069A, 072A, 074A.**

| Pattern ID | Diameter of features | Shape of features | Pitch of features | Array shape | Height of features | Level |
|---|---|---|---|---|---|---|
| 067A | 3 | circles | 6 | triangular | 5 | 1 |
| 068A | 25 | Circles, no flutes | 35 | triangular | 30 | 2 |
| 069A | 35 5 by 5 micron flutes | Circles, flutes | 35 | triangular | 30 | 2 |
| 072A | 300 | Undulated circles | 300 | triangular | 100 | 3 |
| 074A | 067A+069A+072 A (3+35+300) | Stacked 3 level | 6+35+300 | triangular multi level | 85 total as fabricated | 1,2,3 |

As noted herein above, L1 or level 1 correlates with third set of micro features 20, L2 or level 2 correlates with second set of micro features 14, L3 or level 3 correlates with first set of micro features 12, and "flutes" correlates with fourth set of micro features 24.

Friction force for these patterns is shown in Table 4. All of the patterns increased sliding friction. The addition of flutes to a uniform pattern of pillars increased the sliding force as did height of the micro features. These results are for the individual layers that were also combined into hierarchical structures.

**Table 4 - Friction force of micro patterns 067A, 068A, 069A and 074A against moist meat.**

| Texture | Moist meat (grams force) |
|---|---|
| 067AH, Flat, 3µm diameter circles (PLA) N=10 | 14 +/- 3 |
| 068AH, Flat, 25 µm diameter circles (PLA) N=10 | 24 +/- 5 |
| 069AH, Flat, 35µm dia. fluted circles (PLA) N=10 | 52 +/- 8 |
| 074A, (PLA) N =10 | 118+/-12 |
| 074A, (PLA) N =10 trial 2 | 127 +/-18 |

The sliding friction performance of pattern 074A equaled or exceeded that of the natural red rose petal surface. Additional patterns were made as shown in Table 5.

**Table 5 - Description of micro patterns 086A, 089A and 095A**

| Pattern ID | Diameter of features | Shape of features | Pitch of features | Array shape | Height of features | Level |
|---|---|---|---|---|---|---|
| 086A | 3+35 5 by 5 micron 12 zflutes | Circles Flutes | 6+35 | triangular | 4+45 | 1,2 |
| 089A | 750 | Circle undulated | 750 | triangular | 500 | 3 |
| 095A | 086A+089A 3+35+750 | circles | 6+35+750 | triangular | 45, 370 | 1,2,3 |

Pattern 095A was tested for friction as shown in Table 6. Sliding force substantially exceeds that of the natural rose petal surface.

**Table 6 - Friction force of micro pattern 095A against moist meat.**

| Texture | Moist meat (grams force) |
|---|---|
| 095A, (PLA) N=10 | 185 +/-19 |

The surface area of pattern 095A is calculated as shown in Table 7.

**Table 7 - Calculated surface area of pattern 095A.**

| **Feature** | **Added surface area mm² /100 mm²** |
|---|---|
| Level 3 undulated | 213 |
| Level 2 fluted pillars | 686 |
| Level 1 pillars | 279 |
| Total | 1179 |

Together the second and third sets of micro features (level 2 and level 1 as noted in Table 7) substantially increase the surface area exposed to the fluid covering the opposite surface from substrate 10 as shown in Table 7.

### Pattern Designs:

85A - Combination of L1 and L2
   L1:3 micron circular pillars, 6 micron pillar pitch, 5 micron pillar depth.
   L2: 25 micron circular pillars, 35 micron pillar pitch, 30 micron pillar depth, includes flutes 3 micron flute width, 6 micron flute pitch, 5 micron flute depth
086A - Stacked, Fluted, undulated:
   L1: 25 µm circular holes, 35 µm pitch, 45 µm depth
   Includes flutes 3 µm wide, 6 µm pitch, 5 µm deep
   L2: 3 µm circular holes, 6 µm pitch, 5 µm depth
   L3: Flat substrate
087A - L3: 450 micron undulated, 450 micron pitch, 300 micron depth
088A - L3: 600 micron undulated, 600 micron pitch, 400 micron depth
089A - L3: 750 micron undulated, 750 micron pitch, 500 micron depth
090A - Combination of pattern 085A and 087A (L3 300 micron undulation depth - actual was 90 microns deep); actual means the actual depth of the undulation on the mold
091A - Combination of pattern 085A and 088A (L3 400 micron undulation depth - actual was 160 microns deep)
092A - Combination of pattern 085A and 089A (L3 500 micron undulation depth - actual was 205 microns deep)
093A - Stacked, Fluted, undulated:
   L2: 25 µm circular holes, 35 µm pitch, 45 µm depth Includes flutes 3 µm wide, 6 µm pitch, 5 µm deep
   L1:3 µm circular holes, 6 µm pitch, 5 µm depth
   L3 Undulating Background: 450 µm undulated holes, 450 µm pitch,
   300 µm depth (actual depth measured at -200 µm)
094A - Stacked, Fluted, undulated:
   L2: 25 µm circular holes, 35 µm pitch, 45 pm depth Includes flutes 3 pm wide, 6 µm pitch, 5 µm deep
   L1:3 pm circular holes, 6 µm pitch, 5 µm depth
   L3 Undulating Background: 600 µm undulated holes, 600 µm pitch,
   400 µm depth (actual depth measured at -205 µm)
095A - Stacked, Fluted, undulated:
   L2: 25 µm circular cavities, 35 µm pitch, 45 µm depth Includes flutes 3 µm wide, 6 µm pitch, 5 µm deep
   L1:3 µm circular cavities, 6 µm pitch, 5 µm depth
   L3 Undulating Background: 750 µm undulated cavities, 750 µm pitch,
   500 µm depth (actual depth measured at -240 µm)

Pitch is defined as spacing from center to center between microstructures. Work on additional L2 pattern reveled that making flutes deeper, they intersect and thus you need to use few flutes. This helps balance out for added surface area. However the calculations revealed some other options. Adding more flutes of the same size, increasing pillar height from 30 to 45 microns and changing to a closer packed triangular packing have a big effect on added surface area.

The undulating surface pattern 95A detailed above initially produced a friction force of 185 gr/cm², which is a substantial improvement in the art. Based on the data provided in earlier testing, gram forces in sliding friction testing increases with fluted/ribbed structures (fourth set of micro features 24 disposed on side surface of second set of micro features 14). Further, increasing depth at the L3 undulation (first set of micro features 12) increased gram forces, and thus surface adhesion on wet tissue. Later testing and refinement proved this accurate with pattern 95A reaching 325 gr/cm².

The natural rose petal was the inspiration for pattern 095A. However, there are key differences that allow pattern 095A to be more superhydrophobic and have a greater adhesive property. The rose petal has two hierarchical tiers, which allow for a combination of the standard wetting regimes. These two layers also increase the surface area of the rose petal. Pattern 095A has three tiers with added flutes and/or ribs on the sides of the second set of micro features 14, giving the surface even more area, which increases the contact area interface and adhesive force between the liquid substrate and the material surface. The geometry of the first, second and third sets of micro features 12, 14, 20 also increases the roughness of the surface, making it rougher than the rose petal. The rounded undulating surface features of said first set of micro features 12 resemble the rounded papillae seen on the surface of the natural rose petal. However, the papillae on the rose petal seem to be placed in a random fashion, while the undulations on pattern 095A are in specific and predictable locations. An obvious advantage of pattern 095A is that it can be molded onto any polymeric surface.

Preferably, the undulating surface forming first set of micro features 12 comprises a shape selected from the group consisting of rounded sloping projections and rounded sloping cavities forming rounded peaks and rounded valleys that produce a continuously curving surface on at least a portion of said substrate.

In one preferred embodiment, the undulating surface of substrate 10 has a sinusoidal waveform cross-section. In a further embodiment, a pitch between each of the rounded sloping projections and each of the rounded sloping cavities of the undulating surface is within a range of about 450 microns to about 750 microns to facilitate adhesion of said substrate against a liquid covered surface. Additionally, in a further embodiment, a diameter at a generally circular base of each of said rounded sloping projections and each of said rounded sloping cavities of the undulating surface is within a range of about 450 microns to about 750 microns to facilitate adhesion of said substrate against a liquid covered surface. Further, a height/depth of each of said rounded sloping projections and each of said rounded sloping cavities of the undulating surface is within a range of about 100 microns to about 500 microns to facilitate adhesion of said substrate against a liquid covered surface.

Preferably, a pitch between each of said second set of micro features 14 is within a range of about 10 microns to about 50 microns to facilitate adhesion of said substrate against a liquid covered surface. A diameter of each of said second set of micro features is within a range of about 10 microns to about 50 microns to facilitate adhesion of said substrate against a liquid covered surface. A height/depth of each of said second set of micro features is within a range of about 10 microns to about 50 microns to facilitate adhesion of said substrate against a liquid covered surface.

Preferably, a pitch between each of said third set of micro features 20 and each of said forth set of micro features 24 is within a range of about 1 microns to about 10 microns to facilitate adhesion of said substrate against a liquid covered surface. A diameter of each of said third set of micro features 20 and each of said forth set of micro features 24 is within a range of about 0.4 microns to about 10 microns to facilitate adhesion of said substrate against a liquid covered surface. A height/depth of each of said third set of micro features 20 and each of said forth set of micro features 24 is within a range of about 0.4 microns to about 10 microns to facilitate adhesion of said substrate against a liquid covered surface.

### Manufacturing Process:

The specific compression molding process used to create pattern 095A is superior to existing methods used to create surfaces exhibiting the petal effect. In addition to the template, the only material needed is the polymer to be molded. This process is done in one easy step, making the method both time effective and cost effective, while leaving minimal room for error. Pattern 095A surface also more closely mimics the rose petal by having the largest features rounded. Other fabricated imitations all have sharp edges and rectangular geometries. More importantly, the micro surface can be printed onto any polymer using the sample template. This is critical in order for the product to be used for many applications across multiple industries. Pattern 095A is currently the only "rose petal effect" micro surface that can be easily printed on any polymer using compression molding with no additional steps, materials, or coatings needed.

The second and third sets of micro features 14, 20 are made by standard lithographic etching processes well known to those skilled in the art. For example these structures are made by fabricating masks and then etching the patterns into silicon wafers. The micro feature patterns may then be transferred to polymer films by methods well known to those skilled in the art (See US Pat. No. 8,720,047; 8,814,954; US Pub. No. 2011/0311764; US Pub. No. 2012/0043693; US Pub. No. 2012/0052241; and, US Pub. No. 2012/0126458).

The second and third sets of micro feature 14, 20 patterns on the previously described polymer films are combined with first set of micro features 12 by molding methods well known to those skilled in the art and as detailed herein below. (See also, US Pub. No. 2011/0266724).

The fabrication method includes fabricating a first microstructured prototype comprising micro features having a preselected pattern of pillars and/or cavities using stereolithography or using additive manufacturing methods (rapid prototypes / 3D printing).

A rubber sheet is then cast from the first microstructured prototype, thereby making a microstructured rubber sheet comprising rubber micro features having the preselected pattern; this cast rubber is formed as a thin rubber sheet of thickness 10 microns to 3000 microns thick.

A second microstructured prototype is fabricated comprising micro features having a preselected pattern of a series of undulating surface shapes forming peaks and valleys using additive manufacturing (rapid prototypes / 3D printing).

A rubber sheet is cast from the second microstructured prototype, thereby making a microstructured rubber comprising the negative image of the second micro structured prototype on the cast rubber.

A rubber sheet is cast from the rubber cast from the second micro structured prototype thus creating a rubber positive and negative of the second microstructured prototype.

An oxygen plasma treatment is applied to one surface of the rubber positive or negative of the second microstructured prototype to create a highly chemically reactive surface.

A fluoro-silane is bonded to the highly chemically reactive surface to render this surface chemically inert and highly lubricious.

The rubber sheet is then compression molded from the first microstructured prototype between the rubber positive and negative of the second microstructured prototype thereby elastically stretching the sheet over the undulating surface shapes of the rubber positive and negative of the second microstructured prototype and chemically bonding the sheet to the non-siliane treated surface. This forms a rubber mold with the micro features of the first microstructured prototype oriented normal over the surface of the undulating surface features of the second micro structured prototype.

This rubber mold may be used to mold other thermoplastic or other thermoset polymers in a single compression molding step; or it may be used as a mandrel to electroform nickel or copper metal surfaces; or it may be used to mold powdered metals mixed with polymer or wax binders.

The electroformed nickel or copper metal pieces may be used as a tool to form polymers or they may be used as an electrode for electrical discharge machining to machine the shapes into steel and other durable metals and ceramics.

The powdered metals mixed polymer or wax binders may be sintered to form steel or stainless steel metal parts with the micro features of the first microstructured prototype oriented normal over the surface of the undulating surface features of the second micro structured prototype.

## Claims

1. A microstructured surface comprising:
a substrate (10) having an undulating surface defining a first set of micro features (12);
a second set of micro features (14) disposed on said first set of micro features (12) and selected from the group consisting of microstructured projections and microstructured cavities, and combinations thereof, wherein said second set of micro features (14) is smaller than said first set of micro features (12);
a third set of micro features (20) disposed on at least one of said first set of micro features (12) and said second set of micro features (14), said third set of micro features (20) selected from the group consisting of microstructured projections and microstructured cavities, and combinations thereof, wherein said third set of micro features (20) is smaller than said second set of micro features (14);
said second set and said third set of micro features (14, 20) each including a portion of micro features that extend along an axis normal to the curve of said undulating surface;
**characterized by**:
a fourth set of micro features (24) disposed on side surfaces of each microstructure of said second set of micro features (14), wherein said fourth set of micro features (24) is selected from the group consisting of flutes and ribs;
wherein each of said sets of micro features (12, 14, 20, 24) cooperate to increase the surface area and affect at least one of adhesion, friction, hydrophilicity and hydrophobicity of said substrate (10).

2. The microstructured surface of claim 1, wherein each microstructure of said first, second, third and fourth sets of micro features (12, 14, 20, 24) has a respective pitch, height/depth, and diameter, and wherein said micro features are arranged so that liquids penetrate between at least said first and second sets of micro features (12, 14) in a Wenzel fully wetted state when applied against a liquid covered surface with a water droplet greater than 10 micro liters in size.

3. The microstructured surface of claim 1, wherein said undulating surface defining said first set of micro features (12) includes rounded peaks (15) that facilitate pressure distribution across said substrate (10).

4. The microstructured surface of claim 3, wherein said second and third sets of micro features (14, 20) are uniformly distributed across said rounded peaks (15) to provide increased surface area to said first set of micro features(12).

5. The microstructured surface of claim 4, wherein said rounded peaks (15) define areas of increased pressure when said substrate (10) is applied against a liquid covered surface that promote a transition of liquid droplets from a suspended Cassie-Baxter state to a Wenzel fully wetted state among at least said first and second sets of micro features (12, 14).

6. The microstructured surface of any one of the preceding claims, wherein said undulating surface comprises a shape selected from the group consisting of rounded sloping projections and rounded sloping cavities forming rounded peaks (15) and rounded valleys (17) that produce a continuously curving surface on at least a portion of said substrate (10).

7. The microstructured surface of claim 6, wherein a pitch between each of said rounded sloping projections (15) and each of said rounded sloping cavities (17) of said undulating surface is within a range of about 450 microns to about 750 microns to facilitate adhesion of said substrate (10) against a liquid covered surface.

8. The microstructured surface of claim 6, wherein a diameter at a generally circular base of each of said rounded sloping projections (15) and each of said rounded sloping cavities (17) of said undulating surface is within a range of about 450 microns to about 750 microns to facilitate adhesion of said substrate (10) against a liquid covered surface.

9. The microstructured surface of claim 6, wherein a height/depth of each of said rounded sloping projections (15) and each of said rounded sloping cavities (17) of said undulating surface is within a range of about 100 microns to about 500 microns to facilitate adhesion of said substrate (10) against a liquid covered surface.

10. The microstructured surface of any one of the preceding claims, wherein a pitch between each of said second set of micro features (14) is within a range of about 10 microns to about 50 microns to facilitate adhesion of said substrate (10) against a liquid covered surface.

11. The microstructured surface of any one of the preceding claims, wherein a diameter of each of said second set of micro features (14) is within a range of about 10 microns to about 50 microns to facilitate adhesion of said substrate (10) against a liquid covered surface.

12. The microstructured surface of any one of the preceding claims, wherein a height/depth of each of said second set of micro features (14) is within a range of about 10 microns to about 50 microns to facilitate adhesion of said substrate (10) against a liquid covered surface.

13. The microstructured surface of any one of the preceding claims wherein a pitch between each of said third set of micro features (20) and each of said fourth set of features (24) is within a range of about 1 microns to about 10 microns to facilitate adhesion of said substrate (10) against a liquid covered surface.

14. The microstructured surface of any one of the preceding claims, wherein a height/depth of each of said third set of micro features (20) and each of said fourth set of micro features (14) is within a range of about 0.4 microns to about 10 microns to facilitate adhesion of said substrate (10) against a liquid covered surface.

## Patentansprüche

1. Mikrostrukturierte Oberfläche, umfassend:
ein Substrat (10) mit einer welligen Oberfläche, die einen ersten Satz Mikromerkmale (12) definiert;
einen zweiten Satz Mikromerkmale (14), der an dem ersten Satz Mikromerkmale (12) angeordnet ist und ausgewählt ist aus der Gruppe bestehend aus mikrostrukturierten Vorsprüngen und mikrostrukturierten Hohlräumen und Kombinationen davon, wobei der zweite Satz Mikromerkmale (14) kleiner als der erste Satz Mikromerkmale (12) ist;
einen dritten Satz Mikromerkmale (20), der an mindestens einem des ersten Satzes Mikromerkmale (12) und des zweiten Satzes Mikromerkmale (14) angeordnet ist, wobei der dritte Satz Mikromerkmale (20) ausgewählt ist aus der Gruppe bestehend aus mikrostrukturierten Vorsprüngen und mikrostrukturierten Hohlräumen und Kombinationen davon, wobei der dritte Satz Mikromerkmale (20) kleiner ist als der zweite Satz Mikromerkmale (14);
wobei der zweite Satz und der dritte Satz Mikromerkmale (14, 20) jeweils einen Abschnitt Mikromerkmale beinhalten, die sich entlang einer Achse normal zur Kurve der welligen Oberfläche erstrecken;
**gekennzeichnet durch**:
einen vierten Satz Mikromerkmale (24), die an Seitenflächen von jeder Mikrostruktur des zweiten Satzes Mikromerkmale (14) angeordnet sind, wobei der vierte Satz Mikromerkmale (24) ausgewählt ist aus der Gruppe bestehend aus Rillen und Rippen;
wobei jeder der Sätze Mikromerkmale (12, 14, 20, 24) kooperieren, um die Oberfläche zu erhöhen und um mindestens eines aus Adhäsion, Reibung, Hydrophilie und Hydrophobie des Substrats (10) zu beeinflussen.

2. Mikrostrukturierte Oberfläche nach Anspruch 1, wobei jede Mikrostruktur des ersten, zweiten, dritten und vierten Satzes Mikromerkmale (12, 14, 20, 24) eine entsprechende Neigung, Höhe/Tiefe und einen Durchmesser hat, und wobei die Mikromerkmale angeordnet sind, sodass Flüssigkeiten zwischen mindestens dem ersten und zweiten Satz Mikromerkmale (12, 14) in einem vollständig benetzten Wenzel-Zustand eindringen, wenn sie gegen eine mit Flüssigkeit bedeckte Oberfläche mit einem Wassertropfen aufgetragen werden, der größer als 10 Mikroliter ist.

3. Mikrostrukturierte Oberfläche nach Anspruch 1, wobei die wellige Oberfläche, die den ersten Satz Mikromerkmale (12) definiert, abgerundete Spitzen (15) beinhaltet, die die Druckverteilung über das Substrat (10) erleichtern.

4. Mikrostrukturierte Oberfläche nach Anspruch 3, wobei der zweite und dritte Satz Mikromerkmale (14, 20) gleichmäßig über die abgerundeten Spitzen (15) verteilt sind, um eine vergrößerte Oberfläche zu dem ersten Satz Mikromerkmale (12) bereitzustellen.

5. Mikrostrukturierte Oberfläche nach Anspruch 4, wobei die abgerundeten Spitzen (15) Bereiche mit erhöhtem Druck definieren, wenn das Substrat (10) auf eine mit Flüssigkeit bedeckte Oberfläche aufgetragen wird, die einen Übergang von Flüssigkeitstropfen von einem schwebenden Cassie-Baxter-Zustand zu einem vollständig benetzten Wenzel-Zustand aus mindestens dem ersten und zweiten Satz Mikromerkmale (12, 14) fördern.

6. Mikrostrukturierte Oberfläche nach einem der vorhergehenden Ansprüche, wobei die wellige Oberfläche eine Form umfasst, ausgewählt aus der Gruppe bestehend aus abgerundeten, geneigten Vorsprüngen und abgerundeten, geneigten Hohlräumen, die abgerundete Spitzen (15) und abgerundete Täler (17) bilden, die eine kontinuierlich gekrümmte Oberfläche an mindestens einem Abschnitt des Substrats (10) erzeugen.

7. Mikrostrukturierte Oberfläche nach Anspruch 6, wobei eine Neigung zwischen jedem der abgerundeten, geneigten Vorsprünge (15) und jedem der abgerundeten, geneigten Hohlräume (17) der welligen Oberfläche in einem Bereich von etwa 450 Mikron bis etwa 750 Mikron ist, um die Adhäsion des Substrats (10) an einer mit Flüssigkeit bedeckten Oberfläche zu vereinfachen.

8. Mikrostrukturierte Oberfläche nach Anspruch 6, wobei ein Durchmesser an einer im Allgemeinen kreisförmigen Basis von jedem der abgerundeten, geneigten Vorsprünge (15) und jedem der abgerundeten, geneigten Hohlräume (17) der welligen Oberfläche in einem Bereich von etwa 450 Mikron bis etwa 750 Mikron ist, um die Adhäsion des Substrats (10) an einer mit Flüssigkeit bedeckten Oberfläche zu vereinfachen.

9. Mikrostrukturierte Oberfläche nach Anspruch 6, wobei eine Höhe/Tiefe von jedem der abgerundeten, geneigten Vorsprünge (15) und jedem der abgerundeten, geneigten Hohlräume (17) der welligen Oberfläche in einem Bereich von etwa 100 Mikron bis etwa 500 Mikron ist, um die Adhäsion des Substrats (10) an einer mit Flüssigkeit bedeckten Oberfläche zu vereinfachen.

10. Mikrostrukturierte Oberfläche nach einem der vorhergehenden Ansprüche, wobei eine Neigung zwischen jedem des zweiten Satzes Mikromerkmale (14) in einem Bereich von etwa 10 Mikron bis etwa 50 Mikron ist, um die Adhäsion des Substrats (10) an einer mit Flüssigkeit bedeckten Oberfläche zu vereinfachen.

11. Mikrostrukturierte Oberfläche nach einem der vorhergehenden Ansprüche, wobei ein Durchmesser von jedem des zweiten Satzes Mikromerkmale (14) in einem Bereich von etwa 10 Mikron bis etwa 50 Mikron ist, um die Adhäsion des Substrats (10) an einer mit Flüssigkeit bedeckten Oberfläche zu vereinfachen.

12. Mikrostrukturierte Oberfläche nach einem der vorhergehenden Ansprüche, wobei eine Höhe/Tiefe von jedem des zweiten Satzes Mikromerkmale (14) in einem Bereich von etwa 10 Mikron bis etwa 50 Mikron ist, um die Adhäsion des Substrats (10) an einer mit Flüssigkeit bedeckten Oberfläche zu vereinfachen.

13. Mikrostrukturierte Oberfläche nach einem der vorhergehenden Ansprüche, wobei eine Neigung zwischen jedem des dritten Satzes Mikromerkmale (20) und jedem des vierten Satzes Mikromerkmale (24) in einem Bereich von etwa 1 Mikron bis etwa 10 Mikron ist, um die Adhäsion des Substrats (10) an einer mit Flüssigkeit bedeckten Oberfläche zu vereinfachen.

14. Mikrostrukturierte Oberfläche nach einem der vorhergehenden Ansprüche, wobei eine Höhe/Tiefe von jedem des dritten Satzes Mikromerkmale (20) und jedem des vierten Satzes Mikromerkmale (14) in einem Bereich von etwa 0,4 Mikron bis etwa 10 Mikron ist, um die Adhäsion des Substrats (10) an einer mit Flüssigkeit bedeckten Oberfläche zu vereinfachen.

## Revendications

1. Surface microstructurée comprenant :
un substrat (10) ayant une surface ondulée définissant un premier ensemble de micro-caractéristiques (12) ;
un deuxième ensemble de micro-caractéristiques (14) disposé sur ledit premier ensemble de micro-caractéristiques (12) et sélectionné parmi le groupe constitué de saillies microstructurées et de cavités microstructurées, et d'associations de celles-ci, dans laquelle ledit deuxième ensemble de micro-caractéristiques (14) est plus petit que ledit premier ensemble de micro-caractéristiques (12) ;
un troisième ensemble de micro-caractéristiques (20) disposé sur au moins un dudit premier ensemble de micro-caractéristiques (12) et dudit deuxième ensemble de micro-caractéristiques (14), ledit troisième ensemble de micro-caractéristiques (20) étant sélectionné parmi le groupe constitué de saillies microstructurées et de cavités microstructurées, et d'associations de celles-ci, dans laquelle ledit troisième ensemble de micro-caractéristiques (20) est plus petit que ledit deuxième ensemble de micro-caractéristiques (14) ;
ledit deuxième ensemble et ledit troisième ensemble de micro-caractéristiques (14, 20) incluant chacun une partie de micro-caractéristiques qui s'étendent le long d'un axe normal à la courbe de ladite surface ondulée ;
**caractérisé par** :
un quatrième ensemble de micro-caractéristiques (24) disposé sur des surfaces latérales de chaque microstructure dudit deuxième ensemble de micro-caractéristiques (14), dans laquelle ledit quatrième ensemble de micro-caractéristiques (24) est sélectionné parmi le groupe constitué de cannelures et de nervures ;
dans laquelle chacun desdits ensembles de micro-caractéristiques (12, 14, 20, 24) coopèrent pour augmenter la superficie et affecter au moins un parmi l'adhérence, le frottement, l'hydrophilicité et de l'hydrophobicité dudit substrat (10).

2. Surface microstructurée selon la revendication 1, dans laquelle chaque microstructure dudit premier, deuxième, troisième et quatrième ensembles de micro-caractéristiques (12, 14, 20, 24) a un pas, une hauteur/profondeur, et un diamètre respectif, et dans laquelle lesdites micro-caractéristiques sont agencées pour que des liquides pénètrent entre au moins lesdits premier et deuxième ensembles de micro-caractéristiques (12, 14) dans un état complètement mouillé de Wenzel lors de l'application contre une surface couverte de liquide avec une gouttelette d'eau dont la taille est supérieure à 10 microlitres.

3. Surface microstructurée selon la revendication 1, dans laquelle ladite surface ondulée définissant ledit premier ensemble de micro-caractéristiques (12) inclut des pics arrondis (15) qui facilitent la distribution de pression sur ledit substrat (10).

4. Surface microstructurée selon la revendication 3, dans laquelle lesdits deuxième et troisième ensembles de micro-caractéristiques (14, 20) sont uniformément distribués sur lesdits pics arrondis (15) pour fournir une superficie accrue audit premier ensemble de micro-caractéristiques (12).

5. Surface microstructurée selon la revendication 4, dans laquelle lesdits pics arrondis (15) définissent des zones de pression accrue, lorsque ledit substrat (10) est appliqué contre une surface couverte de liquide, qui favorisent une transition de gouttelettes de liquide d'un état de Cassie-Baxter suspendu à un état complètement mouillé de Wenzel parmi au moins lesdits premier et deuxième ensembles de micro-caractéristiques (12, 14).

6. Surface microstructurée selon l'une quelconque des revendications précédentes, dans laquelle ladite surface ondulée comprend une forme sélectionnée parmi le groupe constitué de saillies en pente arrondies et de cavités en pente arrondies formant des pics arrondis (15) et des creux arrondis (17) qui produisent une surface s'incurvant en continu sur au moins une partie dudit substrat (10).

7. Surface microstructurée selon la revendication 6, dans laquelle un pas entre chacune desdites saillies en pente arrondies (15) et chacune desdites cavités en pente arrondies (17) de ladite surface ondulée est au sein d'une plage d'environ 450 microns à environ 750 microns pour faciliter l'adhérence dudit substrat (10) contre une surface couverte de liquide.

8. Surface microstructurée selon la revendication 6, dans laquelle un diamètre à une base généralement circulaire de chacune desdites saillies en pente arrondies (15) et chacune desdites cavités en pente arrondies (17) de ladite surface ondulée est au sein d'une plage d'environ 450 microns à environ 750 microns pour faciliter l'adhérence dudit substrat (10) contre une surface couverte de liquide.

9. Surface microstructurée selon la revendication 6, dans laquelle une hauteur/profondeur de chacune desdites saillies en pente arrondies (15) et de chacune desdites cavités en pente arrondies (17) de ladite surface ondulée est au sein d'une plage d'environ 100 microns à environ 500 microns pour faciliter l'adhérence dudit substrat (10) contre une surface couverte de liquide.

10. Surface microstructurée selon l'une quelconque des revendications précédentes, dans laquelle un pas entre chacune dudit deuxième ensemble de micro-caractéristiques (14) est au sein d'une plage d'environ 10 microns à environ 50 microns pour faciliter l'adhérence dudit substrat (10) contre une surface couverte de liquide.

11. Surface microstructurée selon l'une quelconque des revendications précédentes, dans laquelle un diamètre de chacune dudit deuxième ensemble de micro-caractéristiques (14) est au sein d'une plage d'environ 10 microns à environ 50 microns pour faciliter l'adhérence dudit substrat (10) contre une surface couverte de liquide.

12. Surface microstructurée selon l'une quelconque des revendications précédentes, dans laquelle une hauteur/profondeur de chacune dudit deuxième ensemble de micro-caractéristiques (14) est au sein d'une plage d'environ 10 microns à environ 50 microns pour faciliter l'adhérence dudit substrat (10) contre une surface couverte de liquide.

13. Surface microstructurée selon l'une quelconque des revendications précédentes, dans laquelle un pas entre chacune dudit troisième ensemble de micro-caractéristiques (20) et chacune dudit quatrième ensemble de caractéristiques (24) est au sein d'une plage d'environ 1 micron à environ 10 microns pour faciliter l'adhérence dudit substrat (10) contre une surface couverte de liquide.

14. Surface microstructurée selon l'une quelconque des revendications précédentes, dans laquelle une hauteur/profondeur de chacune dudit troisième ensemble de micro-caractéristiques (20) et de chacune dudit quatrième ensemble de micro-caractéristiques (14) est au sein d'une plage d'environ 0,4 microns à environ 10 microns pour faciliter l'adhérence dudit substrat (10) contre une surface couverte de liquide.
